(19) Europäisches Patentamt
European Patent Office
Office européen des brevets

(11) Publication number: **0 145 191**
**A2**

(12) # EUROPEAN PATENT APPLICATION

(21) Application number: 84307240.6

(22) Date of filing: 19.10.84

(51) Int. Cl.[4]: C 07 C 85/18
C 07 C 87/28

(30) Priority: 10.11.83 US 550347

(43) Date of publication of application:
19.06.85 Bulletin 85/25

(84) Designated Contracting States:
BE DE FR GB IT NL

(71) Applicant: TEXACO DEVELOPMENT CORPORATION
2000 Westchester Avenue
White Plains New York 10650(US)

(72) Inventor: Alexander, David Christopher
4900 Black Angus Cove
Austin Texas 78759(US)

(72) Inventor: Knifton, John Frederick
10900 Catskill Trail
Austin Texas 78750(US)

(74) Representative: Brock, Peter William et al,
Michael Burnside & Partners 2 Serjeants' Inn Fleet Street
London EC4Y 1HL(GB)

(54) **A process for selective preparation of secondary and tertiary aralkyl amines.**

(57) Secondary and tertiary aralkylamines can be prepared with good yield and selectivity by reacting an aromatic vinylic olefin, a nitrogen-containing compound, and synthesis gas comprising carbon monoxide and hydrogen (or in an alternative embodiment, CO and $H_2O$), in the presence of a catalyst comprising a ruthenium-containing compound and an amide solvent at a temperature of at least 100°C and a pressure of at least 1.45 MPa.

Linear products are obtained preferentially under these conditions, but when a quarternary phosphonium compound is added, branched products are also obtained in good yields.

EP 0 145 191 A2

# A PROCESS FOR SELECTIVE PREPARATION OF SECONDARY AND TERTIARY ARALKYL AMINES

This invention relates to an aminomethylation process, and more particularly this invention relates to the selective preparation of secondary and tertiary aralkylamines from aromatic vinylic olefins (a preferred example of which is styrene), primary or secondary amines, and synthesis gas, in the presence of a catalyst system comprising a ruthenium-containing compound, and heating the mixture in the presence of an amide solvent at a temperature of at least 100°C and a pressure of at least 1.45 MPa, until there is substantial formation of the desired secondary or tertiary aralkylamine, and optionally separating said amine product by a novel phase separation technique. The resulting aralkylamines are useful as surfactant type amines.

The principle of obtaining amines, using olefins, hydrogen or water, carbon monoxide, and a primary or secondary amine as reactants, is known. Various techniques have been described embodying this principle and using catalysts of various kinds.

US-A-3 234 283 discloses a process for producing tertiary amines, in better yields and at lower pressures than previously used, by treating CO, hydrogen and a secondary amine with a $C_{10}$-$C_{13}$ olefinic mixture in the presence of a cobalt carbonyl-trialkylphosphine catalyst. Olefin conversion was 88% and there was 48% yield of n-($C_{11}$-$C_{14}$)alkyldimethylamine in a resulting solid fraction from the catalyst.

US-A-3 513 200 covers the utilization of Group VIII metal complexes bearing a biphyllic ligand, such as a phosphine, and these complexes may optionally contain a metal hydride complexed with carbon monoxide. Poly(heterocyclo)amines can be added as an adjuvant. The reaction is carried out at a temperature between 50°and 200°C and under a pressure ranging from 0.5 to 35 MPa. A significant

proportion of aldehydes is obtained, and the selectivity to amines in this process is still only very moderate.

A paper by Iqbal in Helvetica Chimica Acta, Volume 54, pages 1440 to 1445 (1971), as well as US-A-3 947 458 describe the catalytic aminomethylation of olefins, employing a rhodium oxide catalyst, an iron carbonyl catalyst, and a mixed rhodium oxide/iron carbonyl catalyst. (Rhodium carbonyls form during the reaction.) The overall reaction is as follows:

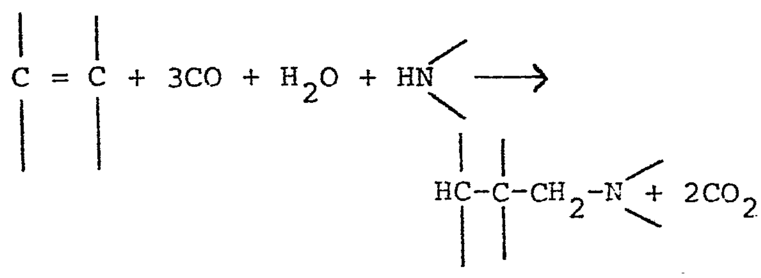

(1)

The mixed rhodium/iron carbonyl catalyst of Iqbal is said to be superior to the rhodium carbonyl catalyst alone, and to the iron carbonyl catalyst alone. This mixed rhodium carbonyl/iron carbonyl catalyst is, however, believed by Laine (US-A-4 292 242) to be less selective. It also has other disadvantages, among which are the following: the rhodium carbonyl/iron carbonyl catalyst is not stable and is prone to decompose; its use results in carboxy amide by-products; and it reduces some of the intermediate aldehyde to an alcohol. Also, in the case of an olefin having a terminal vinyl group ($-CH=CH_2$), a considerable proportion of the amino product has a branched chain,

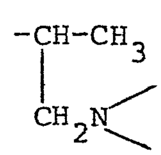

rather than a straight chain,

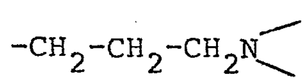

The straight chain products are more important commercially.

US-A-4 096 150 discloses a process for the manufacture of tertiary amines wherein an olefin, hydrogen, CO,

and a secondary amine, are reacted in the presence of a coordination complex catalyst of a Group VIII metal and a ligand, whose donor atom is oxygen, nitrogen or sulphur.

In J. Org. Chem. 45 3370 (1980), Laine et al. describe the results of their studies on the aminomethylation reaction using a variety of Group VIII transition-metal carbonyl catalyst precursors.

US-A-4 292 242 states that the object of its invention is to provide improved methods of aminomethylation which are more selective and lead to fewer unwanted by-products, such as alcohols and carboxy amides. A further object was to provide a more stable mixed carbonyl catalyst, the use of which would result in higher yields of the desired amines. Here, the claimed catalyst is a mixed ruthenium carbonyl/iron carbonyl in a suitable solvent. Again, this process leads to a formamide by-product.

In J. Org. Chem., 47, 445 (1982), Jachimowicz et al. discuss various attempts to devise a one-step, efficient and general conversion of olefins to amines. Among the catalysts used by various workers have been iron pentacarbonyl, rhodium oxide, ruthenium/iron carbonyl and iridium catalysts. The article discusses the feasibility of various aminomethylation syntheses.

In prior art aminomethylation processes, the reaction must often take place at high temperatures and/or pressures, the olefin conversion and selectivity to the desired tertiary amines are not as high as desired, unwanted by-products such as formamides are often formed, and a high degree of desired linearity is not achieved. Additionally, separation of the desired product from by-products is often difficult, expensive and time consuming: separation by distillation can be relatively difficult because of the high boiling point of the products. None of these processes attempts in particular to produce secondary and tertiary aralkylamines which can be used to provide surfactant type amines.

Another concept not approached in the references

discussed, is a means to control distribution of the products, including whether branched or linear. Of course better selectivity is always a desired goal. Finally, none of the processes discussed provides an efficient means for separating the catalyst from the product.

It would be a considerable advance in the art to devise a system for producing aralkylamines, usable as surfactant amines, from vinyl olefins such as substituted or unsubstituted styrene, synthesis gas (i.e. carbon monoxide and hydrogen) and primary and secondary amines by an aminomethylation process wherein the selectivity of production of branched and linear amine could be controlled, the distribution of various amines in the product mixture could be controlled by choice of solvent, and the conversion and selectivity figures would indicate an improvement over prior processes. In addition, it would be an advance over prior art to devise a process which proceeds under milder reaction conditions, forms fewer unwanted by-products, such as formamides, and affords easy and efficient separation of the desired product from by-products and catalyst.

These and other desirable results are achieved by the process of this invention wherein secondary and tertiary aralkylamines are prepared by an aminomethylation process which comprises reacting an aromatic vinylic olefin (e.g. styrene or a substituted styrene), a nitrogen-containing compound (e.g. a primary or secondary amine), and synthesis gas (a mixture of carbon monoxide and hydrogen or, in a modified embodiment CO and $H_2O$), in the presence of a catalyst system comprising a ruthenium-containing compound, optionally mixed with a quaternary phosphonium salt, and in the presence of an amide solvent, and heating the resulting mixture at a temperature of at least 100°C and a pressure of at least 1.45 MPa until there is substantial formation of the desired secondary or tertiary aralkylamine.

In the presence of certain added solvent media, the

desired secondary or tertiary amine product can be separated from the ruthenium catalyst by a useful phase separation technique.

In a preferred embodiment of the invention, secondary and tertiary aralkylamines are prepared via the aminomethylation of styrene with primary and secondary amines. The reaction is catalyzed by a ruthenium catalyst and may be carried out in amide solvents.

In this preferred invention to prepare secondary and tertiary aralkylamines from styrene, synthesis gas ($CO/H_2$) and primary or secondary amines, the syntheses may be represented by the following general equation (2).

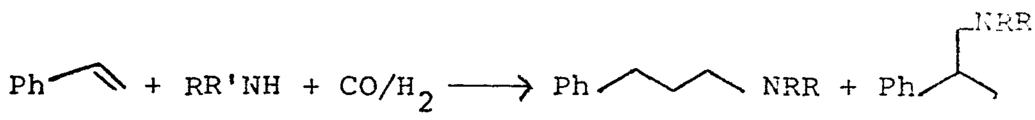 (2)

(A) (B)

wherein R and R' are hydrocarbyl groups containing 1 to 20 carbon atoms, or alternatively, either R or R' (but not both) may be hydrogen.

Here, the improvements over the prior art provided by the present invention include:

a) Improved selectivity to linear product (A).

b) Improved control over the product mixture, whether it be preferably linear (A) or branched (B).

c) Suppression of competing side-reactions, such as reduction of the substrate styrene to ethylbenzene.

d) Ease of separation of the ruthenium catalyst from the desired secondary and tertiary amine products A and B.

It is a surprising feature of this invention, for example, that the selectivity to linear amine (A) can be relatively high. In previous, related reactions of styrene, catalyzed by transition metal complexes (such as hydroformylation), selectivity to the desired product is

often decreased as a result of undesired hydrogenation of the styrene to ethylbenzene. Additionally, styrene reactions involving addition to the olefinic double bond frequently lead predominantly to branched chain products (B) by Markovnikov addition. In the present invention, the anti-Markovnikov amine product (A) can be obtained in high yields.

If, on the other hand, it is desired in this invention to generate primarily the branched chain amine product (B), then the addition of a phosphonium salt, such as tetrabutylphosphonium bromide, to the ruthenium catalyst, ensures a higher proportion of branched chain amine in the crude liquid product. The composition of the amide solvent used in the present invention may also influence the selectivity between the linear and branched amine products, A and B.

Suppression of competing side-reactions, such as hydrogenation of styrene to unwanted ethylbenzene, is achieved by choice of ruthenium catalyst solvent. In the case of aminomethylation of styrene with primary amine coreactants, yield of secondary amine product may in some cases be lowered by competing carbonylation of the amine to formamide.

The use of an amide solvent allows more efficient separation of secondary or tertiary amine product from the ruthenium catalyst. When certain amide solvents, such as hydroxyethylpyrrolidone, are used, aminomethylation produces two-phase mixtures that allow easy separation of most of the catalyst components from the desired amine products.

In order to present the inventive concept in the greatest possible detail and to promote its understanding, the following supplementary disclosure is submitted.

A. CATALYST COMPOSITION

The catalyst components suitable in the practice of this invention essentially include a ruthenium-containing compound, optionally in the presence of an aliphatic or

aromatic amide solvent, and a quaternary phosphonium salt. The actual catalytically-active species is unknown, but is believed to comprise ruthenium in complex combination with carbon monoxide and hydrogen.

In the practice of this invention, the ruthenium-containing catalyst may be chosen from a wide variety of organic and inorganic compounds, complexes, etc., as will be shown and illustrated below. It is only necessary that the catalyst precursor actually employed should contain ruthenium in any of its ionic states.

The ruthenium catalyst-precursors may take many different forms. For instance, the ruthenium may be added to the reaction mixture as an oxide, as in the case of for example, ruthenium(IV) oxide hydrate, anhydrous ruthenium-(IV) dioxide and ruthenium(VIII) tetraoxide. Alternatively, it may be added as the salt of a mineral acid, e.g. ruthenium(III) chloride hydrate, ruthenium(III) bromide, ruthenium(III) iodide, tricarbonyl ruthenium(II) iodide, anhydrous ruthenium(III) chloride and ruthenium nitrate, or as the salt of a suitable organic carboxylic acid, for example, ruthenium(III) acetate, ruthenium naphthenate or ruthenium valerate, or as a ruthenium complex with a carbonyl-containing ligand, such as ruthenium(III) acetylacetonate. The ruthenium may also be added to the reaction zone as a ruthenium carbonyl or hydridocarbonyl derivative. Here, suitable examples include triruthenium dodecacarbonyl and other hydridocarbonyls such as $H_2Ru_4(CO)_{13}$ and $H_4Ru_4(CO)_{12}$, and substituted carbonyl species such as the tricarbonylruthenium(II) chloride dimer, $[Ru(CO)_3Cl_2]_2$.

Preferred ruthenium-containing compounds include oxides of ruthenium, and ruthenium carbonyl or hydridocarbonyl derivatives. Among these, particularly preferred are ruthenium(IV) dioxide hydrate, anhydrous ruthenium(IV) oxide and triruthenium dodecacarbonyl.

Especially good results were observed with triruthenium dodecacarbonyl or ruthenium oxide.

The optional quaternary "onium" salt to be used in the catalyst composition may be any onium salt but is preferably one containing phosphorus, such as salts of the formula

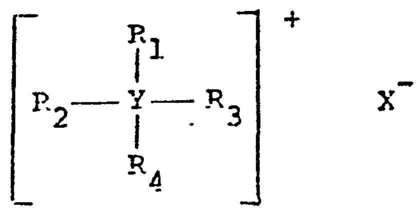

wherein Y is phosphorus, $R_1$, $R_2$, $R_3$ and $R_4$ are organic groups, which may be the same or different, preferably alkyl, aryl or alkaryl, and X is an anion. The organic groups useful in this instance include those alkyl groups having from 1 to 20 carbon atoms in a branched or linear alkyl chain, such as methyl, ethyl, n-butyl, isobutyl, octyl, 2-ethylhexyl and dodecyl. Tetraethylphosphonium bromide and tetrabutylphosphonium bromide are typical examples at present in commercial production. The corresponding quaternary phosphonium acetates, nitrates, chromates, tetrafluoroborates, and other halides, such as the corresponding chlorides and iodides, are also satisfactory.

B. <u>FEEDSTOCK</u>

The feedstock used in the practice of this invention comprises a vinylic olefin, such as styrene or a substituted styrene, a primary or secondary amine, carbon monoxide and hydrogen.

The process can be applied to any aromatic vinylic olefin. Suitable aromatic vinylic olefin substrates having 8 to 25 carbon atoms include styrene, 2,5-dimethylstyrene, 2,4-dimethylstyrene, 2-methylstyrene, 3-methylstyrene, 4-methylstyrene, vinyl naphthalene, α-methylstyrene, divinylbenzene, 2-vinylpyridine, 4-vinylpyridine, 2-methyl-5-vinylpyridine, and 2-vinylimidazole.

Particularly preferred in the practice of this invention is the use of styrene, α-methylstyrene, p-methylstyrene or divinylbenzene.

Suitable nitrogen-containing coreactants useful in

the practice of this invention include primary and secondary amines having 1 to 20 carbon atoms. These amines may be straight or branched chain aliphatic series, they may be cycloaliphatic amines, or they may be aromatic amines. Examples of suitable primary aliphatic and aromatic amines include methylamine, ethylamine, n-propylamine, n-hexylamine, n-dodecylamine and aniline. Suitable cycloaliphatic amines include cyclohexylamine. Secondary aliphatic amines that are satisfactory coreactants include dimethylamine, diethylamine, methylethylamine, di(n-propyl)amine, di(isopropyl)amine, di(ethylhexyl)amine, piperidine, morpholine, di(n-heptyl)amine, and di(n-decyl)amine, as well as 2-aminooctane, N-methylaniline, pyrrolidine and 2-hydroxyethyl-pyrrolidone. Aliphatic diamines such as piperazine are also useful in the practice of this invention. Ammonia is a less effective coreactant.

The quantity of nitrogen-containing coreactant employed in the present invention is not critical, and may vary over a wide range. In general, however, it is desirable to conduct the reaction in the presence of sufficient primary or secondary amine to satisfy the stoichiometry of equation (1).

C. SYNTHESIS GAS

The relative amounts of carbon monoxide and hydrogen which can be initially present in the synthesis gas mixture are variable over a wide range. In general, the mol ratio of $CO:H_2$ is preferably in the range from 20:1 to 1:20, and more preferably from 5:1 to 1:5, although ratios outside these ranges may also be employed with good results. Particularly in continuous operations, but also in batch operations, the carbon monoxide-hydrogen gaseous mixtures may also be used in conjunction with up to 50% by volume of one or more other gases. These other gases may include one or more inert gases such as nitrogen, argon or neon, or they may include gases that may or may not undergo reaction under carbon monoxide hydrogenation conditions, such as carbon dioxide, and hydrocarbons, such as methane,

ethane or propane.

Aminomethylation of aromatic, vinylic olefins may also be practiced using carbon monoxide alone as the gas, when in conjunction with controlled quantities of water. This modification of the invention is illustrated in Example 13.

D. SOLVENT

The reaction is carried out in the presence of a suitable amide solvent, (e.g. an aliphatic amide or aromatic amide). If such solvents were to be omitted, and with styrene as substrate, ethylbenzene formation is a major side reaction, separation of the amine product from the ruthenium catalyst is difficult, and there is less control over the distribution of linear-to-branched secondary and tertiary amine products, (A and B in equation 2). These solvents should be liquids under the conditions of the aminomethylation reactions of equation 2, and should substantially solubilize the ruthenium compound catalyst components.

The important advantages of the preferred solvent are enhanced yields of desired amine, improved amine product linearity, and ease of separation of the desired secondary and tertiary amine product from the ruthenium catalyst through the formation of two-phase liquid products. The Examples will demonstrate that the use of solvent leads to improvements in both the yield and desired linearity or branched structure of the desired amine.

Suitable amide solvents may be N,N-dimethylformamide, N,N-dimethylacetamide, N-hydroxyethylpyrrolidone, N-methylpyrrolidone, N-isopropylpyrrolidone, N,N-diethylformamide, N,N-dimethylacetamide, N,N-dimethylbenzamide, N,N-diphenylformamide, N,N-dimethylbutyramide and N-benzylpyrrolidone.

A preferred class of solvents for aminomethylation of styrene includes N,N-dimethylformamide, N,N-dimethylacetamide, and N-substituted pyrrolidones such as N-isopropylpyrrolidone and N-hydroxyethylpyrrolidone. The best

solvent for obtaining linear amines appears to be N-hydroxyethylpyrrolidone (HEP).

Some of the particular advantages noted when adding N-hydroxyethylpyrrolidone (HEP) solvent to the reaction mixture include:

1. The conversion and selectivity to linear tertiary amines are both higher with HEP as solvent than in its absence as will be demonstrated by Examples 5, 12 and 16 in comparison to other examples.

2. The liquid product cleanly separates into two phases, the upper layer of which is rich in amine product; in the lower layer HEP predominates, but unreacted primary or secondary amine and soluble ruthenium catalyst are also present. These layers are easily separated. The lower layer, rich in catalyst, may be recycled.

The amount of solvent employed may be varied as desired. In general, it is desirable to use sufficient solvent to fluidize the catalyst system.

E. CONCENTRATION

The quantity of ruthenium compound, quaternary phosphonium salt and amide solvent employed in the present invention is not critical, and may vary over a wide range. In general, the novel process is desirably conducted in the presence of a catalytically effective quantity of active ruthenium species, in quaternary onium salt, and optionally a solvent which gives the desired product in reasonably yield. The reaction proceeds when employing as little as $1 \times 10^{-6}$ weight % and even lesser amounts, of ruthenium.

The upper concentration is dictated by a variety of factors including catalyst cost, partial pressure of carbon monoxide and hydrogen, operating temperature, etc. A ruthenium concentration of from 0.0001 to 1 weight % in conjunction with a quaternary onium concentration of from 0.001 to 10 weight %, and a solvent concentration of from 10 to 70 weight %, based on the total weight of the reaction mixture is desirable in the practice of this invention.

F. TEMPERATURE

The temperature range which can usefully be employed in the process of the invention may vary over a considerable range depending upon experimental factors, including choice of catalyst, pressure and other variables. The process can take place at from 100 to 300°C or more. The preferred temperatures are 120°C and more preferably from 120 to 220°C. Coming under special consideration are temperatures from 150 to 190°C.

G. PRESSURE

Superatmospheric pressures of 0.8 MPa or greater lead to substantial yields of the desired amines. A preferred range is from 1.45 to 41.5 MPa; although pressures above 41.5 MPa also provide useful yields of the desired products. A preferred range is from 3.5 to 14.0 MPa.

The pressures referred to herein represent the total pressure generated by all the reactants, although they are substantially due to the carbon monoxide and hydrogen reactants.

H. BY-PRODUCTS

The desired products of the reaction, the secondary and tertiary aralkyl amines are formed in significant quantities varying from 30 to 90% yields. Also formed will be minor by-products, such as ethyl benzene, and formamides. The desired secondary and tertiary aralkyl amine products can be recovered from the reaction mixture by conventional means, such as fractional distillation in vacuo, etc., but distillation could be relatively difficult in some instances because of the high boiling points of the products.

Further, in the process of this invention it has been discovered that an amide solvent, such as N-hydroxyethylpyrrolidone can be added to the reaction mixture to increase selectivity to the desired linear products. This process allows for a useful phase separation technique, which works well in separating the product amines from the ruthenium-onium-solvent catalyst. The crude reaction

product mixture consists of two layers and the product amine is isolated from the upper, catalyst-free layer.

I. INTRODUCTION OF CATALYST

The process of the invention can be conducted in a batchwise, semi-continuous or continuous manner. The catalyst can be initially introduced into the reaction zone batchwise, or it may be continuously or intermittently introduced into such a zone during the course of the synthesis reaction. Operating conditions can be adjusted to optimize the formation of the desired linear secondary or tertiary amines, and said material can be recovered by methods known to the art, such as distillation, fractionation and extraction. A fraction rich in the catalyst components may then be recycled to the reaction zone, if desired, and additional amine product generated.

J. IDENTIFICATION TECHNIQUES

The products have been identified in this work by one or more of the following analytical procedures; viz, gas-liquid chromatography (glc), infrared (ir), mass spectometry, nuclear magnetic resonance (nmr) and elemental analyses, or a combination of these techniques. All temperatures are in degrees centigrade and all pressures in Megapascals (MPa).

Yields of secondary and tertiary alkyl amine products have been estimated on the basis of the quantity of aromatic vinyl olefin converted, and expressed as a percentile. The figures are estimated from gas-liquid chromatography data.

The linearity of the amine products has been estimated by measuring the quantity of linear amine formed (A) in accordance with equation 1, divided by the total secondary or tertiary amine formed. Linearity is also expressed as a percentage.

To illustrate the process of the invention, the following examples are given. It is to be understood, however, that the examples are given in the way of illustration are are not to be regarded as limiting the invention

in any way.

EXAMPLE 1

Example 1 illustrates the synthesis of phenylpropylamines from styrene, diethylamine and synthesis gas with hydroxyethylpyrrolidone added to ensure ease of separation of the phenylpropylamine products from the ruthenium catalyst.

Styrene (10 ml, 87 mmol), diethylamine (10 ml, 97 mmol), trirutheniumdodecacarbonyl (0.1g 0.156 mmol), and hydroxyethylpyrrolidone (10 ml) were added to a 300 ml stirred autoclave, which was then sealed and flushed with 1:1 $CO/H_2$. After pressurization to 3.55 MPa, the reaction mixture was heated to 152°C and stirred for 5.5 hours, with one repressurization to 4.95 MPa. The reactor was then cooled and the two-phase product was withdrawn. Both layers had a volume of 15 ml; the upper layer was clear and light yellow while the lower layer was clear and dark red. Analysis by glc showed the following compositions:

| | -Product Composition (%)- | |
|---|---|---|
| | upper | lower |
| diethylamine | 11% | 11% |
| ethylbenzene | 6 | 2 |
| phenylpropylamines | 72 | 24 |
| linearity | 63 | 67 |
| HEP | 11 | 53 |

Estimated conversion of styrene in this Example is: 91%.
Estimated yield of total phenylpropylamines is: 92%.
Ruthenium recovery in the upper and lower layers is:
Lower Layer 4230 ppm Upper Layer 9 ppm.

EXAMPLES 2 to 6

Examples 2 to 6 illustrate the aminomethylation of styrene following the procedures of Example 1, but employing different primary and secondary amine coreactants, and different ruthenium catalyst precursors. Results are summarized in Table 1,

It may be noted that:

1) $RuO_2$ may be used in place of $Ru_3(CO)_{12}$ (Example 2).

2) Primary and secondary cyclic amines are suitable coreactants (Examples 3 to 5).

TABLE 1[a]

| Example | Amine | t(h) | -Product Composition (%)[e]- 1 | 2 | 3 | 4 | Product Amine Lin. | Product Composition Layers |
|---|---|---|---|---|---|---|---|---|
| 2[b] | $Et_2NH$ | 5 | 14/17 | 7/3 | 63/21 | 11/57 | 65/70 | 15/15 |
| 3[c] | $tBuNH_2$ | 6.5 | 8 | 5 | 36 | 51 | 61 | |
| 4 | pip | 6 | 0/0 | 5/0 | 87/19 | 5/74 | 60/66 | 13/13 |
| 5 | morph | 4.5 | 0/0 | 9/6 | 52/31 | 29/43 | 59/61 | 4/14 |
| 6[d] | Et NH | 6 | 5 | 3 | 46 | 44 | 60 | |

a. Reactions were run with styrene and the indicated amine at 150°C and 3.55 - 4.95 MPa 1:1 $CO/H_2$. The compositions given are those of the two product layers and are in the order upper/lower; layers gives the volume in ml of each layer. Lin refers to linearity of the product amines, pip is piperazine, and morph is morpholine.

b. Catalyst was $RuO_2$ instead of $Ru_3(CO)_{12}$.

c. The product of this Example consisted of only one phase.

d. The solvent was DMF; only one phase in the product.

e. Products were identified as follows:
   1. $Et_2NH$ or other starting amines
   2. Ethylbenzene
   3. Total product amine, linear + branched, e.g. in Example 2.

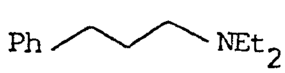 plus 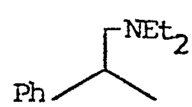

   4. Hydroxyethylpyrrolidone (HEP), except in Example 6 (DMF).

EXAMPLES 7 and 8

Examples 7 and 8 show that inferior results may be obtained without amide solvents. In Example 7, no solvent was used, and in Example 8 tetraethylene glycol methyl ester was the solvent.

TABLE 2[a]

| Example | Amine | t(h) | T(C) | Products (%) 1 | 2 | 3 | 4 | Amine lin |
|---|---|---|---|---|---|---|---|---|
| 7 | $tBuNH_2$ | 3 | 155 | 8 | 31 | 45 | - | 39 |
| 8 | $Et_2NH$ | 5 | 145 | 9 | 7 | 27 | 43 | 51 |

a. Identification as in Table 1.

EXAMPLE 9

In Example 9, α-methylstyrene was the aromatic vinylic olefin coreactant. The reaction was carried out with 11 ml (85 mmol) of α-methylstyrene and 10 ml (97 mmol) of diethylamine with 10 ml of dimethylacetamide as solvent. The catalyst was $Ru_3(CO)_{12}$ (0.1g, 0.156 mmol), and tetrabutylphosphonium bromide (1.0g, 2.9 mmol) was added. The reaction was carried out as described above at 4.95 MPa and 165°C for 6.5 hours. The product was a clear red solution which gave the following analysis:

| | |
|---|---|
| diethylamine | 23% |
| cumene | 2 |
| α-methylstyrene | 25 |
| dimethylacetamide | 39 |
| amine product | 8 |

The amine product was identified by its nmr spectrum as (3-phenylbutyl)diethylamine.

EXAMPLE 10

Ammonia was the amine coreactant in Example 10. The reaction was carried out as described in Example 1 with styrene (14 ml, 122 mmol) and ammonia (4g, 235 mmol) in 12 ml of hydroxyethylpyrrolidone with $Ru_3(CO)_{12}$ as catalyst (0.1g, 0.156 mmol) at 4.95 - 5.62 MPa and 147°C for 4.5 hours. Only about 20% of the styrene reacted; ethylbenzene was the major product, and low yields of amine products were formed.

EXAMPLE 11

In Example 11, recycling of the ruthenium catalyst is demonstrated. The lower layer from Example 1 was separated and charged to a 300 ml stirred autoclave with 10 ml (86 mmol) of styrene and 10 ml (97 mmol) of diethylamine. The reaction was carried out at 156°C and 3.55 - 4.95 MPa for 5.5 hours. The following results were obtained:

| | Upper | Lower |
|---|---|---|
| diethylamine | 16% | 27% |
| ethylbenzene | 8 | 2 |
| phenylpropylamines | 63 | 11 |
| linearity | 61 | 61 |
| HEP | 8 | 49 |

EXAMPLE 12

Example 12 shows that the selectivity of the reaction can be changed so that more of the branched product is produced. Reactants were the same as those in Example 1, except that tetrabutylphosphonium bromide (1.2g, 3.5 mmol) was added and DMF was the solvent. The reaction was carried out at 3.89 - 5.27 MPa, with 1:2 $CO/H_2$ and at 155°C for 4 hours. The single-phase product had the following analysis.

| | |
|---|---|
| diethylamine | 9% |
| ethylbenzene | 5 |
| phenylpropylamines | 38 |
| linearity | 44 |
| HEP | 48 |

EXAMPLE 13

In Example 13, the use of the ruthenium catalyst of the Example 12 is illustrated with styrene, secondary amine, and $CO/H_2O$ instead of $CO/H_2$. To a 300 ml stirred autoclave was added styrene (10 ml, 87 mmol), diethylamine (20 ml, 193 mmol), water (4 ml, 222 mmol), DMF (10 ml), $Ru_3(CO)_{12}$ (0.1g, 0.156 mmol), and tetrabutylphosphonium bromide (1.2g, 3.5 mmol). The reaction was carried out at 157°C with 4.95 MPa CO for 6.5 hours. The turbid red

product solution had the following composition.

| | |
|---|---|
| diethylamine | 23% |
| ethylbenzene | 2 |
| phenylpropylamines | 20 |
| linearity | 44 |
| diethylformamide | 13 |
| dimethylformamide | 26 |

It can be seen that in this case diethylformamide is a major by-product.

CLAIMS:

1. A process for preparing secondary and tertiary aralkylamines which comprises reacting an aromatic vinylic olefin, a nitrogen-containing compound, and synthesis gas comprising carbon monoxide and hydrogen, in the presence of a catalyst, characterized in that the catalyst comprises a ruthenium-containing compound, that an amide solvent is employed and that a temperature of at least 100°C and a pressure of at least 1.45 MPa is also employed.

2. A process according to Claim 1 characterized in that the aromatic vinylic olefin has 8 to 25 carbon atoms.

3. A process according to Claim 1 or 2 characterized in that the nitrogen-containing compound is a primary or secondary amine.

4. A process according to any preceding Claim characterized in that the ruthenium-containing compound is an oxide of ruthenium, a ruthenium salt of a mineral acid, a ruthenium salt of an organic carboxylic acid, a ruthenium complex with a carbonyl-containing ligand, a ruthenium carbonyl or hydridocarbonyl or a substituted species thereof.

5. A process according to any preceding Claim characterized in that an aromatic or aliphatic amide solvent is used.

6. A process according to Claim 5 characterized in that the amide solvent is N-isopropylpyrrolidone, N-hydroxyethylpyrrolidone, N-methyl pyrrolidone or N,N-dimethylformamide.

7. A process according to any preceding Claim characterized in that the temperature is from 100 to 300°C.

8. A process according to any preceding Claim characterized in that the pressure is from 1.45 to 41.5 MPa.

9. A process according to any preceding Claim characterized in that the reaction is conducted in the presence of a quaternary phosphonium salt.

10. A modification of the process claimed in any preceding Claim characterized in that the synthesis gas comprises CO and $H_2O$.